# EUROPEAN PATENT SPECIFICATION

(11) **EP 2 554 149 B1**
(45) Date of publication and mention of the grant of the patent: **04.01.2017**
(21) Application number: 11765578.7
(22) Date of filing: 29.03.2011
(51) Int. Cl.: A61F 13/496

(54) **DISPOSABLE ARTICLE FOR WEARING**
EINWEGKLEIDUNGSSTÜCK
ARTICLE JETABLE À PORTER

(30) Priority: 31.03.2010 JP 2010084215
(43) Date of publication of application: 06.02.2013
(73) Proprietor: Unicharm Corporation, Ehime 799-0111 (JP)
(72) Inventor: KATSURAGAWA, Kunihiko, Kanonji-shi Kagawa 769-1602 (JP); SASAYAMA, Kenichi, Kanonji-shi Kagawa 769-1602 (JP); ICHIKAWA, Makoto, Kanonji-shi Kagawa 769-1602 (JP)
(74) Representative: Peter, Julian
(86) International application number: PCT/JP2011/057901
(87) International publication number: WO 2011/125678

(56) References cited:
- JP-A- 2005 021 196
- JP-B2- 4 205 024
- JP-B2- 4 280 187
- JP-Y2- 2 511 428
- US-A1- 2004 158 217
- US-A1- 2005 020 991
- US-A1- 2005 020 991
- US-A1- 2005 177 124

## Description

### {Technical Field}

The present invention relates to disposable wearing articles and, more particularly, to disposable wearing articles such as disposable diapers, disposable toilet-training pants, disposable incontinent pants and disposable sanitary pants each having a liquid-absorbent structure attached to an outer surface of an annular waist panel.

### {Background}

Conventionally, wearing articles including a liquid-absorbent structure attached to an outer surface of an elasticized waist panel are known. For example, JP 2006-51240 A (PTL 1) discloses a disposable wearing article including a liquid-absorbent crotch panel of which a front end segment and a rear end segment are attached to an outer surface of an elasticized waist panel by the intermediary of detachable fastening means arranged intermittently in a waist circumferential direction.

### {Citation List}

### {Patent Literature}

{PTL 1}: JP 2006-51240 A

### {Summary}

### {Technical Problem}

In the wearing article disclosed in PTL 1, the crotch panel is detachably attached to the waist panel and, after used, only the crotch panel soiled with body waste may be disposed of and replaced with a fresh crotch panel. In other words, it is unnecessary to change the waist panel, and therefore such a wearing article is advantageous not only in economic terms but also from the viewpoint that the article may be stably put on the wearer's body without being affected by the physical type and the crotch shape of the wearer.

However, the crotch panel is attached to the outer surface of the waist panel only with use of the fastening means arranged intermittently in the waist circumferential direction and, with such an arrangement, when a large amount of body waste is received by the crotch panel, there is a possibility that the crotch panel's own weight and the wearer's movements might neutralize the fastening effect of the fastening means during use of the wearing article. To avoid the above-mentioned undesirable situation, it may be contemplated to enlarge an area of the fastening means arranged on the crotch panel by which the crotch panel is fastened to the waist panel. In this case, however, a region in the crotch panel which contains the absorbent core will lie on the side of the outer surface of the waist panel and, in consequence, the liquid-absorption performance might be deteriorated. In addition, there is a possibility that bodily fluids might spread to the part of the absorbent core lying on the outer side of the waist panel and eventually might leak out of the wearing article.

An object of the present invention is to provide a disposable wearing article improved so that a crotch panel may be stably fastened to an outer surface of a waist panel and a relatively large amount of body waste may be absorbed and retained without deteriorating absorbing performance of a liquid-absorbent structure in the crotch panel.

### {Solution to Problem}

Some embodiments of the present invention provides a disposable wearing article having a longitudinal direction and a transverse direction being orthogonal to the longitudinal direction, and including:
a skin-facing side;
a non-skin-facing side;
a front waist region;
a rear waist region;
a crotch region extending between the front and rear waist regions;
elasticized waist panels respectively defining the front and rear waist regions; and
a crotch panel coupled to the elasticized waist panel and defining part of the front and rear waist regions and the crotch region.

This invention further includes the following features:
the crotch panel includes a front end portion lying in the front waist region, a rear end portion lying in the rear waist region and a liquid-absorbent structure extending from the crotch region into the front and rear waist regions;
at least one of the front and rear end portions is coupled to the non-skin-facing side of the elasticized waist panel by the intermediary of a bond region including a non-bond region in its midsection as viewed in the transverse direction; and
the elasticized waist panel having the one of the end portions of the crotch panel coupled thereto is formed at a position opposed to the non-bond region with a separable portion extending in the longitudinal direction.

According to one embodiment of this invention, the crotch panel includes a pair of elasticized lateral portions and, with the wearing article put on the wearer's body, a distance between the pair of elasticized lateral portions is enlarged as the elasticized waist panel is stretched in a circumferential direction about the wearer's waist.

According to another embodiment of this invention, the elasticized waist panel having the one of the end portions of the crotch panel coupled thereto is formed at a position opposed to the non-bond region with a plurality of separable portions arranged in the transverse direction.

According to even another embodiment of this invention, the separable portion is a slit extending in the longitudinal direction.

According to yet another embodiment of this invention, the separable portion is perforations extending in the longitudinal direction.

According to still another embodiment of this invention, the separable portion is a notch.

According to further embodiment of this invention, the separable portion is a cutout.

### {Advantageous Effects of Invention}

According to one or more embodiments of this invention, the elasticized waist panel having at least one of the end portions of the crotch panel coupled thereto is formed at a position opposed to the non-bond region with a separable portion extending in the longitudinal direction. With this unique arrangement, the separable portion is outspread in the transverse direction as the diaper is put on the wearer's body and the opening to the underlying body waste collecting space is formed so that body waste flowing into the waist region may be quickly moved into the body waste collecting space. As another unique arrangement, the crotch panel is not bonded to the elasticized waist panel at the intermediate non-bond region and, as an advantageous consequence, it is possible to ensure an absorbing area required for the liquid-absorbent structure.

### {Brief Description of Drawings}

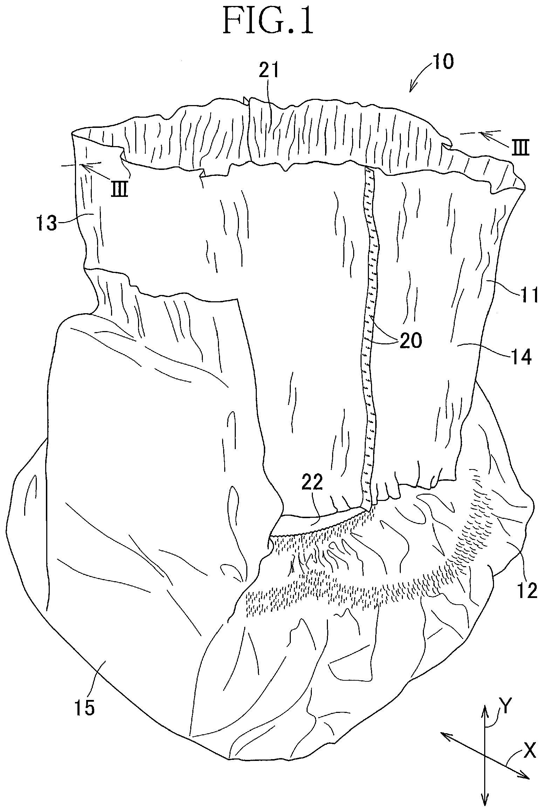

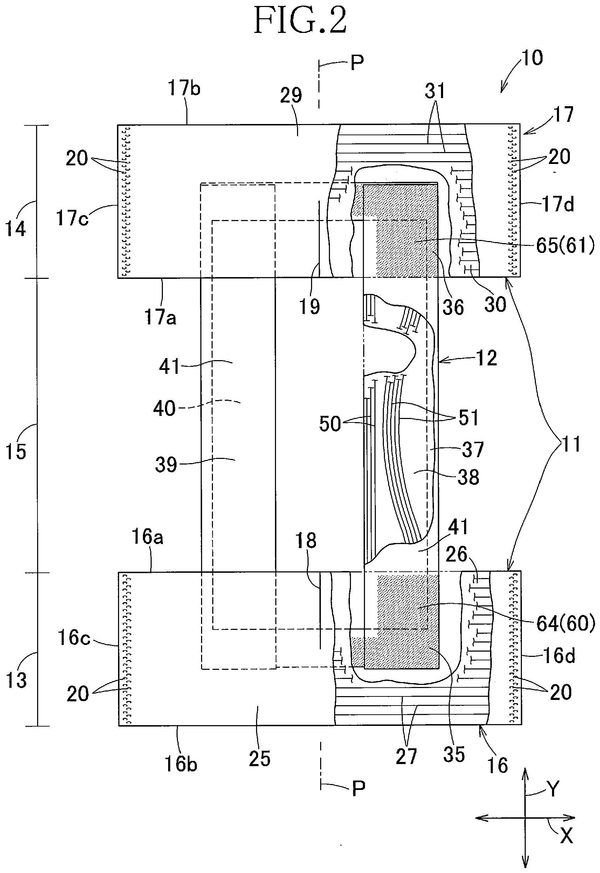

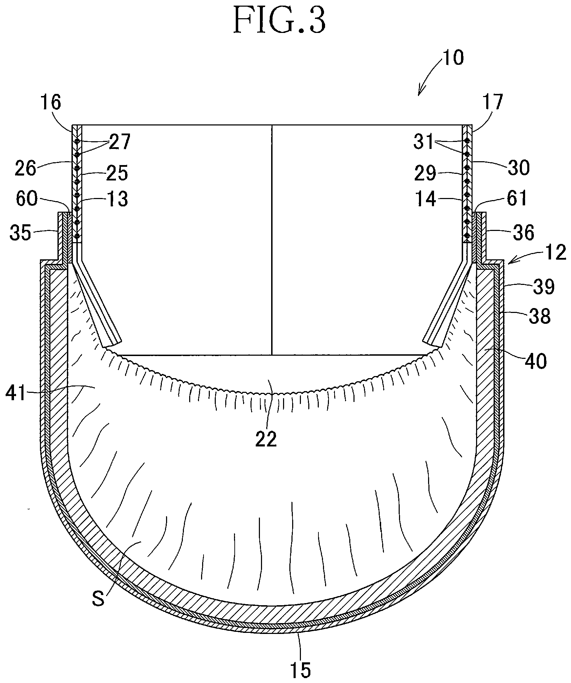

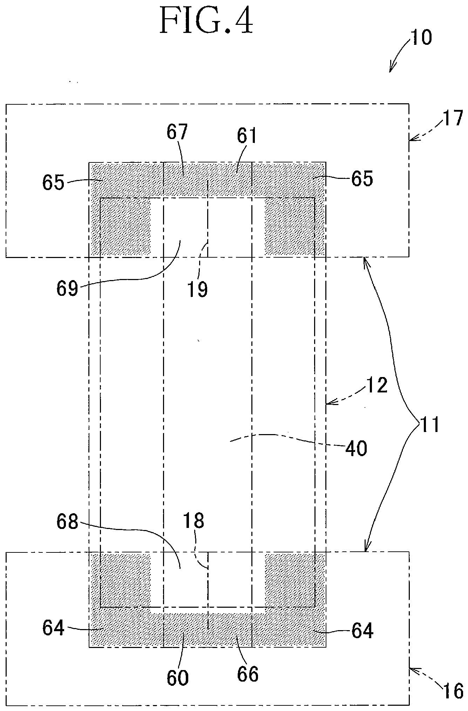

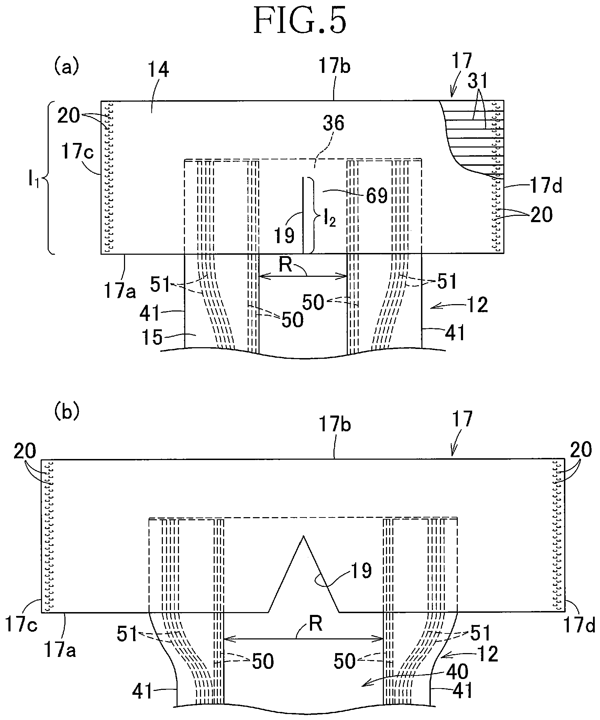

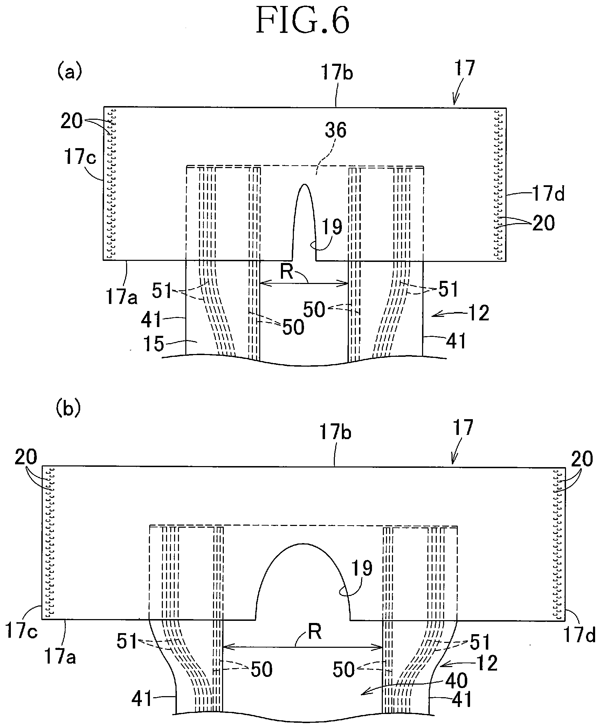

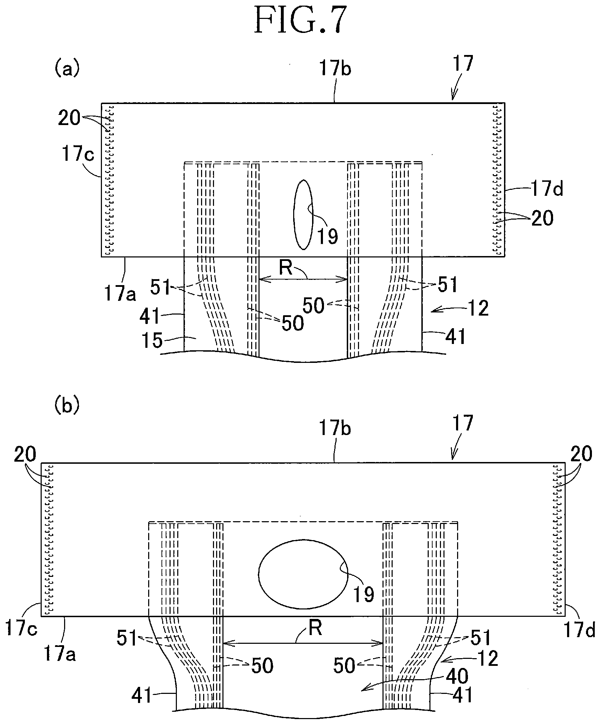

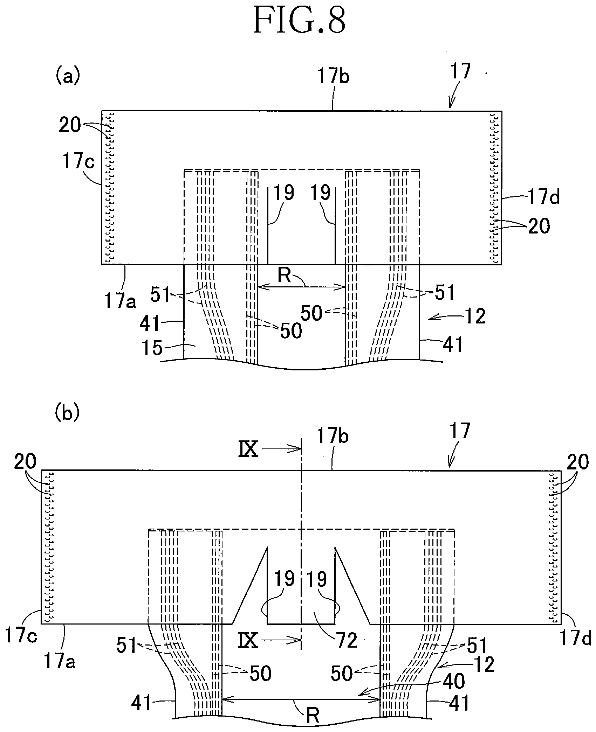

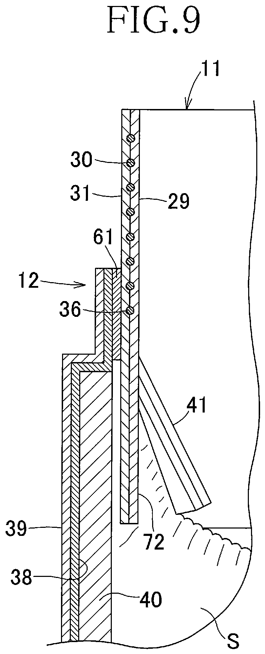

{Fig. 1} Fig. 1 is a perspective view illustrating a disposable diaper as an example of a disposable wearing article according to a first embodiment of this invention.
{Fig. 2} Fig. 2 is a partially cutaway developed plan view of the diaper having seams opened and flatly developed in a front-back direction as viewed from an inner surface thereof.
{Fig. 3} Fig. 3 is a sectional view taken along line III-III in Fig. 1.
{Fig. 4} Fig. 4 is a developed plan view of the diaper to illustrate respective patterns of front and rear bond regions.
{Fig. 5} Fig. 5(a) is a plan view illustrating a front waist region and part of a crotch region of the diaper developed in the front-back direction. Fig. 5(b) is a plan view illustrating the front waist region and part of the crotch region of the diaper put on the wearer's body.
{Fig. 6} Fig. 6(a) is a plan view similar to Fig. 5 (a), illustrating a diaper according to a second embodiment of this invention. Fig. 6(b) is a plan view similar to Fig. 5 (b), illustrating the diaper according to the second embodiment of this invention.
{Fig. 7} Fig. 7 (a) is a plan view similar to Fig. 5 (a), illustrating a diaper according to a third embodiment of this invention. Fig. 7 (b) is a plan view similar to Fig. 5 (b), illustrating the diaper according to the third embodiment of this invention.
{Fig. 8} Fig. 8 (a) is a plan view similar to Fig. 5 (a), illustrating a diaper according to a fourth embodiment of this invention. Fig. 8(b) is a plan view similar to Fig. 5(b), illustrating the diaper according to the fourth embodiment of this invention.
{Fig. 9} Fig. 9 is a sectional view taken along line IX-IX in Fig. 8 (b).

### {Description of Embodiments}

### <First Embodiment>

Fig. 1 is a perspective view illustrating a disposable diaper 10 as an example of a disposable wearing article according to a first embodiment of this invention. Fig. 2 is a partially cutaway developed plan view of the diaper 10 having a pair of side seams 20 opened and flatly developed in a front-back direction as viewed from an inner surface thereof. Fig. 3 is a sectional view taken along line III-III in Fig. 1 and Fig. 4 is a developed plan view of the diaper to illustrate respective patterns of front and rear bond regions. In this regard, an elasticized waist panel and a crotch panel are indicated by imaginary lines in Fig. 4 for convenience of illustration.

Referring to Figs. 1 and 2, the disposable diaper 10 has a longitudinal direction Y and a transverse direction X being orthogonal to the longitudinal direction Y and includes: an imaginary center line P-P bisecting a width dimension in the transverse direction X; a skin-facing side; a non-skin-facing side; an annular elasticized waist panel 11; a crotch panel 12 coupled to the non-skin-facing side of the elasticized waist panel 11; a front waist region 13; a rear waist region 14; and a crotch region 15 extending in the longitudinal direction between the front and rear waist regions 13, 14. The diaper 10 is shaped symmetrically about the imaginary center line P-P, and the elasticized waist panel 11 includes a front waist panel 16 defining the front waist region 13 and a rear waist panel 17 defining the rear waist region 14.

The front waist panel 16 is shaped into a transversely long rectangle defined by an inner end 16a intersecting with the crotch panel 12 and extending in the transverse direction X, an outer end 16b opposed to and spaced from the inner end 16a in the longitudinal direction Y and extending in the transverse direction X, and both lateral edges 16c, 16d extending in the longitudinal direction Y between the inner and outer end edges 16a, 16b. The front waist panel 16 is formed on the imaginary center line P-P thereof with a separable portion 18 defined by a slit extending in the longitudinal direction Y.

The rear waist panel 17 is substantially the same as the front waist panel 16 in shape as well as in size and specifically shaped into a transversely long rectangle defined by an inner end edge 17a intersecting with the crotch panel 12 and extending in the transverse direction X, an outer end edge 17b opposed to and spaced from the inner end edge 17a in the longitudinal direction Y and extending in the transverse direction X, and both lateral edges 17c, 17d extending in the longitudinal direction Y between the inner and outer end edges 17a, 17b. The rear waist panel 17 is formed on the imaginary center line P-P thereof with a separable portion 19 defined by a slit extending in the longitudinal direction Y. While both the front waist panel 16 and the rear waist panel 17 are formed with the separable portions 18, 19 according to this embodiment, only one of the front and rear waist regions 13, 14 may be provided with the separable portion 18 or 19. As will be described in detail later, the formation of the separable portion 18 and/or the separable portion 19 allows a liquid-absorbent structure 40 to ensure a necessary absorption area. For this reason, assuming that the rear waist panel 17 is formed with the separable portion 19, even if the wearer lying on the back or being in a sitting posture excretes, bodily fluids should not leak out of the rear waist region 14. Assuming that the front waist panel 16 is formed with the separable portion 18, when used on a male wearer, the wearer may insert his penis through the separable portion 18 and urinate directly onto the liquid-absorbent structure 40 coupled to the outer surface of the front waist panel 16.In this way, it is possible to prevent urine from leaking out along the front waist panel 16.

The opposite lateral edges 16c, 16d of the front waist panel 16 and the opposite lateral edges 17c, 17d of the rear waist panel 17 are put flat and joined together at side seams 20 intermittently bonding in the longitudinal direction Y so as to define a waist-opening 21 and a pair of leg-openings 22 (See Fig. 1). The side seams 20 may be arranged with use of various known bonding means, e.g., thermal fusion bonding techniques such as a heat embossing process, a sonic process and the like.

The front waist panel 16 has a first inner sheet 25 lying on the skin-facing side and a first outer sheet 26 lying on the non-skin-facing side. The first inner sheet 25 and the first outer sheet 26 are formed of at least one of a liquid-impervious SMS (spunbonded/melt blown/spunbonded) fibrous nonwoven fabric, a spunbonded fibrous nonwoven fabric, a plastic sheet, and a laminate sheet thereof, each of these various kinds of material having a mass per unit area in a range of 15 to 30 g/m². The sheet 25 and the sheet 26 are bonded to each other with a hot melt adhesive (not shown) applied to the inner surface of at least one of these sheets. A plurality of thread, strand or string front waist elastic elements 27 extending in the transverse direction X are interposed between the first inner sheet 25 and the first outer sheet 26 and secured with a hot melt adhesive (not shown) contractibly in the transverse direction X, and the front waist panel 16 may be elasticized at least in the transverse direction X. In this regard, it is also possible to form the front waist panel 16 in such a manner that the first inner sheet 25 and the first outer sheet 26 are bonded to each other only by a hot melt adhesive applied to the respective front waist elastic elements 27.

The rear waist panel 17 has a second inner sheet 29 lying on the skin-facing side and a second outer sheet 30 lying on the non-skin-facing side. The second inner sheet 29 and the second outer sheet 30 are formed of at least one of a liquid-impervious SMS (spunbonded/melt blown/spunbonded)fibrous nonwoven fabric, a spunbonded fibrous nonwoven fabric, a plastic sheet and a laminate sheet thereof, each having a mass per unit area in a range of 10 to 30 g/m². The sheet 29 and the sheet 30 are bonded to each other with a hot melt adhesive (not shown) applied to the inner surface of at least one of these sheets. A plurality of thread, strand or string rear waist elastic elements 31 extending in the transverse direction X are interposed between the second inner sheet 29 and the second outer sheet 30 and secured with a hot melt adhesive (not shown) contractibly in the transverse direction X, and the rear waist panel 16 may be elasticized at least in the transverse direction X. In this regard, it is also possible to form the rear waist panel 17 in such a manner that the second inner sheet 29 and the second outer sheet 30 are bonded to each other only by a hot melt adhesive applied to the respective rear waist elastic elements 31.

The crotch panel 12 is substantially shaped into a rectangle and includes a front end portion 35 coupled to the non-skin-facing side of the front waist panel 16, a rear end portion 36 coupled to the non-skin-facing side of the rear waist panel 17, amidsection 37 extending in the longitudinal direction Y between the front and rear end portions 35, 36, liquid-impervious inner and outer crotch sheets 38, 39, and the liquid-absorbent structure 40 located on the skin-facing side of the inner crotch sheet 38. The inner and outer crotch sheets 38, 39 are bonded to each other with a hot melt adhesive (not shown) applied to the inner surface of one of these two sheets 38, 39 and both lateral margins of these sheets are folded inwardly to define a pair of elasticized lateral portions 41 extending in the longitudinal direction Y on the inner surface of the liquid-absorbent structure 40. The liquid-absorbent structure 40 is obtained by wrapping a semi-rigid absorbent core composed of a mixture of superabsorbent polymer particles and fluff pulp or superabsorbent polymer particles only and having tensile strength lower than that of the respective sheets with a liquid-pervious sheet.

Each of the elasticized lateral portions 41 is provided with a plurality of thread, strand or string first leg elastic elements 50 and second leg elastic elements 51 each extending in the longitudinal direction Y and thereby each of these portions 41 is elasticized at least in the longitudinal direction Y. Specifically, each of the elasticized lateral portions 41 is provided with three first leg elastic elements 50 extending in the longitudinal direction along the inner side edge thereof and five second leg elastic elements 51 curving inwardly in a midsection of the crotch region and rectilinearly extending into the front and rear waist regions 13, 14. The first and second leg elastic elements 50, 51 are secured under tension in the longitudinal direction Y between the inner and outer crotch sheets 38, 39 with a hot melt adhesive (not shown) applied to at least one of the inner and outer crotch sheets 38, 39.

Under the effect of the first and second leg elastic elements 50, 51 respectively provided in the elasticized lateral portions 41, with the diaper 10 put on the wearer's body, the elasticized lateral portions 41 are spaced away from the liquid-absorbent structure 40 and put in contact with the wearer's thighs, and the crotch panel 12 assumes a hammock-like shape slung from the elasticized waist panel 11 (See Figs. 1 and 3) . When body waste is excreted onto the liquid-absorbent structure 40 of the diaper 10 put on the wearer's body, the liquid-absorbent structure 40 is spaced downward from the wearer's buttocks under its own weight so as to define a body waste collecting space S having a volumetric capacity larger than that in conventional disposable diapers between the wearer and the liquid-absorbent structure 40. Such a body waste collecting space S having a relatively large volumetric capacity makes it possible for the diaper 10 to retain a relatively large amount of body waste, and further, since the liquid-absorbent structure 40 is slung from the elasticized waist panel 11 so as to be sufficiently spaced from the wearer's buttocks, the soiling of the wearer's buttocks with body waste may be prevented.

Referring to Figs. 3 and 4, the crotch panel 12 is coupled to the respective outer surfaces of the front and rear waist panels 16, 17 at a front bond region 60 and a rear bond region 61 respectively defined by the front end portion 35 and the rear end portion 36 of the crotch panel 12 coated on the respective non-skin-facing sides of these end portions 35, 36 with a hot melt adhesive. By coupling the front and rear end portions 35, 36 of the crotch panel 12 to the respective non-skin-facing sides of the front and rear waist panels 16, 17, it is possible to secure the body waste collecting space S which has a further large volume. In this regard, only one of the front and rear end portions 35, 36 may be coupled to the non-skin-facing side of the front or rear waist panel 16 or 17 so long as it is possible to ensure the necessary collecting capacity for the body waste collecting space S.

The front and rear bond regions 60, 61 respectively have square U-shapes opening toward the crotch region 15 and include opposite lateral zones 64, 65 defined by segments of the elasticized lateral portions 41 coated with a hot melt adhesive and intermediate zones 66, 67 respectively extending between the opposite lateral zones 64, 65, in the transverse direction X. The intermediate zones 66, 67 are located outboard of a region in which the liquid-absorbent structure 40 is present as viewed in the longitudinal direction Y and the opposite lateral zones 64, 65 and the associated intermediate zones 66, 67 respectively contour non-bond regions 68, 69 which are not coated with a hot melt adhesive. In this regard, the front and rear bond regions 60, 61 each may have a curved shape as a whole and the opposite lateral zones 64, 65 and/or the intermediate zones 66, 67 each may have various shapes such as a stepped shape or a triangular shape.

Fig. 5 (a) is the plan view illustrating the rear waist region 14 and part of the crotch region 15 of the diaper 10 flatly developed in the front-back direction and Fig. 5 (b) is the plan view illustrating the rear waist region 14 and part of the crotch region 15 of the diaper 10 put on a wearer's body. Hereunder, the description will be limited to the arrangement of the rear waist panel 17 because the arrangement of the front waist panel 16 will be redundantly described so long as the front waist panel 16 has an arrangement similar to that of the rear waist panel 17.

Referring to Fig. 5 (a), the rear waist panel 17 is provided with a plurality of the rear waist elastic elements 31 extending in the transverse direction X and contractibly secured thereto to be elasticized in the transverse direction X. Referring to Fig. 5 (b), when the diaper 10 is put on the wearer's body and the elasticized waist panel 11 is stretched in the circumferential direction about the wearer's waist, in other words, the rear waist panel 17 is stretched in the transverse direction X, two sets of the rear waist elastic elements 31 extending between the respective side seams 20 and the separable portion 19 are also stretched since the lateral edges of the rear waist panel 17 are secured by the respective side seams 20. Thereupon the separable portion 19 is outspread in the transverse direction X to form a substantially triangular opening. Though not illustrated, it is also possible to provide the rear waist panel 17 with a plurality of the rear waist elastic elements 31 extending in the transverse direction X on both sides of the separable portion 19 in such a manner that these rear waist elastic elements 31 are not secured to the rear waist panel 17 in the vicinity of the separable portion 19 and non-fixed segments of the elastic elements 31 are cut under tension so as to be cut back.

The separable portion 19 is formed in the region occupied by the liquid-absorbent structure 40 and therefore bodily fluids flowing into the rear waist region 14 is absorbed through the opening of the separable portion 19 by the liquid-absorbent structure 40 underlying this opening (i.e., on the outer surface of the rear waist panel). In this way, the absorption area of the liquid-absorbent structure 40 required to improve the liquid-absorption performance may be secured. It should be noted here that the opening of the separable portion 19 does not deteriorate the bonding strength of the rear end portion of the crotch panel 12 to the rear bond region 61. Body waste having flown on the rear waist region 14 moves under a body waste own weight into the body waste collecting space S via the opening of the separable portion 19 and therefore it is possible to lessen the possibility that body waste clinging to the skin-facing side of the rear waist panel 17 might soil the wearer's skin.

With the diaper 10 put on the wearer's body, the rear waist elastic elements 31 of the rear waist panel 17 cooperate with the first and second leg elastic elements 50, 51 in the respective elasticized lateral portions 41 of the crotch panel 12 to form imaginary elastic zones surrounding the wearer's thighs. The respective elasticized lateral portions 41 are pulled outward in the transverse direction X as the separable portion 19 is outspread to form the opening. In response to the respective elasticized lateral portions 41 being pulled in the transverse direction X, a distance R between the opposite side elasticized portions 41 is correspondingly enlarged, thus the opening of the body waste collecting space S may be enlarged and, at the same time, the jamming of the elasticized lateral portions 41 into the wearer's posterior rugae and/or vicinities thereof may be prevented.

The separable portion 19 may be formed of a line of small halls for tearing, i.e. perforations adapted to be broken when the diaper 10 is put on the wearer's body. In this case, the step of conveying the sheet material web for the rear waist panel 17 will be facilitated compared to when the sheet material web is previously formed with the slits.

If the separable portion 19 extends outward in the longitudinal direction Y beyond the region in which the liquid-absorbent structure 40 is present, a portion of body waste might leak out of the liquid-absorbent structure 40. To avoid such situation, the separable portions 19 may be formed only in the non-bond region 69 in which the crotch panel 12 is not secured to the rear waist panel 17 and the liquid-absorbent structure 40 is present. More specifically, when a length of the lateral edges 17c, 17d of the rear waist panel 17, i.e., a length dimension l₁ of the rear waist region 14 in the longitudinal direction Y is in a range of 150 to 200 mm, a length dimension l₂ of the separable portion 19 in the longitudinal direction Y is preferably in a range of 15 to 150 mm. While the length dimension l₂ of the separable portion 19 may be enlarged by extending the liquid-absorbent structure 40 further outward with respect to the rear waist panel 17, for example, if the length dimension l₁ of the rear waist panel 14 in the longitudinal direction is 115 mm or more, a tensile force of the rear waist panel 17 as a whole in the transverse direction X will be deteriorated and the diaper 10 might not fit the wearer's body. In this regard, these dimensions may be appropriately selected depending on a size of the diaper 10 as a whole and a ratio between these two dimensions, i. e., the length dimension l₂ of the separable portion 19 in the longitudinal direction Y divided by the length dimension l₁ of the rear waist region 14 in the longitudinal direction Y is preferably in a range of about 0.1 to about 0.7.

### <Second Embodiment>

Fig. 6 (a) and Fig. 6 (b) are plan views similar to Fig. 5 (a) and Fig. 5(b), illustrating the diaper 10 according to a second embodiment of this invention.

According to this embodiment, the separable portion 19 of the rear waist panel 17 is not in the form of a straight slit but in the form of an arc-like notch and an open end of the notch is contiguous to the inner end 17a of the rear waist panel 17. When the separable portion 19 in the form of such arc-like notch, an opening serving to guide body waste to the liquid-absorbent structure 40 is previously formed and therefore it is possible to inhibit a tensile force of the rear waist elastic elements 31 compared to the case of the first embodiment. In consequence, when the diaper 10 is put on the wearer's body, the rear waist panel 17 may be easily stretched in the transverse direction by merely pinching the outer end portion 17b by one's fingers and operation to put the diaper on the wearer's body is correspondingly facilitated. In this regard, the notch is not limited to the arc-like shape but may be selected from various known shapes such as a triangular shape and a square shape.

### <Third Embodiment>

Fig. 7 (a) and Fig. 7 (b) are plan views similar to Fig. 5 (a) and Fig. 5(b), illustrating the diaper 10 according to a third embodiment of this invention.

According to this embodiment, the separable portion 19 of the rear waist panel 17 is provided in the form of a vertically long cutout (slot) . The vertically long cutout defining the separable portion is deformed to a circular shape as illustrated in Fig. 7 (b) as the diaper 10 is put on the wearer's body and the rear waist panel 14 is stretched circumferentially about the wearer's waist and bodily fluids pass through this cutout and absorbed by the liquid-absorbent structure 40 lying outboard of this cutout. In this regard, the cutout is not limited to the circular shape but may be selected from various well known shapes such as a triangular shape and a square shape.

### <Fourth Embodiment>

Fig. 8 (a) and Fig. 8 (b) are plan views similar to Fig. 5 (a) and Fig. 5(b), illustrating the diaper 10 according to a fourth embodiment of this invention and Fig. 9 is a sectional view taken along line IX-IX in Fig. 8(b). A basic arrangement of the diaper 10 according to this embodiment is similar to that according to the first embodiment and description will be limited hereunder to features distinguished from those of the first embodiment.

According to this embodiment, an intermediate region of the rear waist panel 17 is formed with a plurality of the separable portions 19. When a plurality of the separable portions 19 are formed in this manner, an intermediate segment 72 defined between each pair of the separable portions 19 is not stretched in the transverse direction X but merely hangs down. In this way, the opening area of the body waste collecting space S is further enlarged and correspondingly larger amount of body waste may be quickly moved into the body waste collecting space S. While the intermediate segment 72 is not provided with the rear waist elastic elements 31 in this embodiment, the intermediate segment 72 also may be provided with these elastic elements 31.

### {Reference Signs List}

10 disposable diaper (disposable wearing article)
11 elasticized waist panel
12 crotch panel
13 front waist region
14 rear waist region
15 crotch region
18, 19 separable portions
35 front end portion of crotch panel
36 rear end portion of crotch panel
40 liquid-absorbent structure
41 elasticized lateral portions
60 front bond region
61 rear bond region
l₁ length dimension of side edges of rear waist region in longitudinal direction
l₂ length dimension of separable portions in longitudinal direction
X transverse direction
Y longitudinal direction

## Claims

1. A disposable wearing article having a longitudinal direction and a transverse direction being orthogonal to the longitudinal direction, the article including:
a skin-facing side;
a non-skin-facing side;
a front waist region (13);
a rear waist region (14);
a crotch region (15) extending between the front and rear waist regions (13,14);
elasticized waist panels (11) respectively defining the front and rear waist regions (13, 14); and
a crotch panel (12) coupled to the elasticized waist panels (11) and defining part of the front and rear waist regions (13, 14) and the crotch region (15), wherein:
the crotch panel (12) includes a front end portion (35) lying in the front waist region (13), a rear end portion (36) lying in the rear waist region (14) and a liquid-absorbent structure (40) extending from the crotch region (15) into the front and rear waist regions (13, 14);
at least one of the front and rear end portions (35, 36) is coupled to the non-skin-facing side of the elasticized waist panel (11) by the intermediary of front and rear bond regions (60, 61) including a non-bond region (68, 69) in its midsection as viewed in the transverse direction; and
the elasticized waist panel (11) having the one of the end portions of the crotch panel (12) coupled thereto is formed at a position opposed to the non-bond region (68, 69) with a separable portion (18, 19) extending in the longitudinal direction, wherein
the front and rear bond regions (60, 61) respectively have square U-shapes opening toward the crotch region (15) and include opposite lateral zones (64, 65) defined by segments of elasticized lateral portions (41) of the crotch panel (12) coated with a hot melt adhesive and intermediate zones (66, 67) respectively extending between the opposite lateral zones (64, 65), in the transverse direction (X).

2. The wearing article according to claim 1, wherein, with the wearing article put on the wearer's body, a distance between the pair of elasticized lateral portions (41) is enlarged as the elasticized waist panel (11) is stretched in a circumferential direction about the wearer waist.

3. The wearing article according to claim 1 or 2, wherein the elasticized waist panel (11) having the one of the end portions of the crotch panel (12) coupled thereto is formed at a position opposed to the non-bond region (68, 69) with a plurality of separable portions (18, 19) arranged in the transverse direction.

4. The wearing article according to any one of claims 1 through 3, wherein the separable portion (18, 19) is a slit extending in the longitudinal direction.

5. The wearing article according to any one of claims 1 through 3, wherein the separable portion (18, 19) is perforation extending in the longitudinal direction.

6. The wearing article according to any one of claims 1 through 3, wherein the separable portion (18, 19) is a notch.

7. The wearing article according to any one of claims 1 through 3, wherein the separable portion (18, 19) is a cutout.

## Patentansprüche

1. Wegwerf Trageartikel mit einer Längsrichtung und einer Querrichtung die orthogonal zu der Längsrichtung ist, der Artikel umfassend:
eine hautzugewandte Seite;
eine nicht-hautzugewandte Seite;
einen vordere Taillenbereich (13);
einen hinteren Taillenbereich (14);
einen Schrittbereich (15), der sich zwischen dem vorderen und dem hinteren Taillenbereich (13, 14) erstreckt;
elastifiizierte Taillenpaneele (11), die jeweils den vorderen und hinteren Taillenbereich (13, 14) bestimmen; und
ein Schrittpaneel (12), das mit den elastifizierten Taillenpaneelen (11) verbunden ist und teilweise vordere und hintere Taillenbereiche (13, 14) und den Schrittbereich (15) bestimmt, wobei:
das Schrittpaneel (12) einen vorderen Endabschnitt (35), der im vorderen Taillenbereich (13) liegt, einen hinteren Endabschnitt (36), der im hinteren Taillenbereich (14) liegt, und eine flüssigkeitsabsorbierende Struktur (40) umfasst,
die sich von dem Schrittbereich (15) in den vorderen und hinteren Taillenbereich (13, 14) erstreckt;
zumindest einer der vorderen und hinteren Endabschnitte (35, 36) mit der nicht-hautzugewandten Seite des elastifizierten Taillenpaneels (11) durch die Zwischenschaltung von vorderen und hinteren Klebebereichen (60, 61), umfassend eines nicht-Klebebereichs (68, 69) in seiner, in Querrichtung gesehenen, Mittelsektion, verbunden ist; und
das elastifizierte Taillenpaneel (11), mit dem der eine der Endabschnitte des Schrittpaneels (12) verbunden ist, an einer Position gegenüber des nicht-Klebebereichs (68, 69) mit einem abtrennbaren Abschnitt (18, 19), der sich in Längsrichtung erstreckt, ausgebildet ist, wobei
der vordere und hintere Klebebereich (60, 61) jeweils winkelige U-Formen haben, die sich in Richtung des Schrittbereichs (15) öffnen und gegenüberliegende seitliche Zonen (64, 65) umfassen, die durch Segmente von elastifizierten seitlichen Abschnitten (41) des Schrittpaneels (12), die mit einem Heißschmelzklebstoff überzogen sind, und Zwischenzonen (66, 67), die sich jeweils zwischen den gegenüberliegenden seitlichen Zonen (64, 65) in die Querrichtung (X) erstrecken, bestimmt werden.

2. Trageartikel nach Anspruch 1, wobei mit dem Trageartikel aufgetragen auf den Körper des Trägers, ein Abstand zwischen dem Paar von elastifizierten seitlichen Abschnitten (41) vergrößert ist, da das elastifizierte Taillenpaneel (11) in eine Umfangsrichtung um die Taille des Trägers gedehnt ist.

3. Trageartikel nach Anspruch 1 oder 2, wobei das elastifizierte Taillenpaneel (11), mit dem der eine der Endabschnitte des Schrittpaneels (12) verbunden ist, an einer Position gegenüber des nicht-Klebabereichs (68, 69) mit einer Vielzahl von abtrennbaren Abschnitten (18, 19), die in Längsrichtung angeordnet sind, ausgebildet ist.

4. Trageartikel nach einem der Ansprüche 1 bis 3, wobei der abtrennbare Abschnitt (18, 19) ein Schlitz ist, der sich in die Längsrichtung erstreckt.

5. Trageartikel nach einem der Ansprüche 1 bis 3, wobei der abtrennbare Abschnitt (18, 19) Perforation ist, der sich in die Längsrichtung erstreckt.

6. Trageartikel nach einem der Ansprüche 1 bis 3, wobei der abtrennbare Abschnitt (18, 19) eine Kerbe ist.

7. Trageartikel nach einem der Ansprüche 1 bis 3, wobei der abtrennbare Abschnitt (18, 19) ein Ausschnitt ist.

## Revendications

1. Article jetable ayant une direction longitudinale et une direction transversale qui est perpendiculaire à la direction longitudinale, l'article comprenant:
un côté faisant face à la peau;
un côté ne pas faisant face à la peau;
une région de ceinture avant (13);
une région de ceinture arrière (14);
une région d'entrejambe (15) se prolongeant entre les régions de ceinture avant et arrière (13, 14);
un panneaux de ceinture élastifiés (11) qui déterminent des régions de ceinture avant et arrière (13, 14); et
un panneaux d'entrejambe (12) qui est relié avec les panneaux de ceinture élastiques (11) et qui détermine partiellement des régions de ceinture avant et
arrière (13, 14) et la région d'entrejambe (15), dans lequel:
le panneaux d'entrejambe (12) comprend une partie d'extrémité avant (35) situé dans la région de ceinture avant (13), une partie d'extrémité arrière (36) se trouvant dans la région de ceinture arrière (14) et une structure absorbant les liquides (40), qui s'étendant de la région d'entrejambes (15) dans les régions de ceinture avant et arrière (13, 14);
au moins une des parties d'extrémité avant et arrière (35, 36) est reliée avec le côté ne pas faisant face à la peau du panneaux de ceinture élastifiés (11) par l'intermédiaire des régions adhésive avant et arrière (60, 61) comprenant une région non-adhésive (68, 69) dans la section centrale vue dans la direction transversale respectivement; et
le panneaux de ceinture élastifiés (11) avec lequel l'une des parties d'extrémité du panneaux d'entrejambe (12) est relié, à une position opposée à la région non-adhésive (68, 69) ayant une partie séparable (18, 19) qui se prolongeant dans la direction longitudinale, dans lequel
la région adhésive avant et arrière (60, 61) ayant chacune les formes en U anguleux qui se débouchent dans la direction de la région d'entrejambe (15) et des zones latérales opposées (64, 65), qui sont déterminés par des segments des parties élastiques latérales (41) du panneaux d'entrejambe (12), qui sont revêtues d'un adhésif thermofusible, et les zones intermédiaires (66, 67), chacun s'étendant entre les zones latérales opposées (64, 65) dans la direction transversale (X).

2. L'article jetable selon la revendication 1, dans lequel une distance entre la paire de parties latérales élastiques (41) est augmentée si l'article vestimentaire est appliqué sur le corps d'un porteur, parce que le panneaux de ceinture élastifiés (11) est étirée dans une direction circonférentielle autour de la taille du porteur.

3. L'article jetable selon la revendication 1 ou 2, dans lequel le le panneaux de ceinture élastifiés (11) avec lequel l'une des parties d'extrémité du panneaux d'entrejambe (12) est relié, est formée à une position opposée à la zone non-adhésive (68, 69) ayant une pluralité de sections séparables (18, 19) qui sont disposés dans la direction longitudinale.

4. L'article jetable selon l'une quelconque des revendications 1 à 3, dans lequel la section détachable (18, 19) est une fissure qui se prolonge dans la direction longitudinale.

5. L'article jetable selon l'une quelconque des revendications 1 à 3, dans lequel la section détachable (18, 19) est une perforation s'étendant dans la direction longitudinale.

6. L'article jetable selon l'une quelconque des revendications 1 à 3, dans lequel la section détachable (18, 19) est une encoche.

7. L'article jetable selon l'une quelconque des revendications 1 à 3, dans lequel la section détachable (18, 19) est une découpe.
